# EUROPEAN PATENT APPLICATION

(11) **EP 2 269 973 A1**
(43) Date of publication of application: **05.01.2011**
(21) Application number: 09735832.9
(22) Date of filing: 07.04.2009
(51) Int. Cl.: C07C 51/255, B01J 27/128, C07C 51/265, C07C 63/307, C07B 61/00

(54) **PROCESS FOR PRODUCTION OF HIGH-PURITY TRIMELLITIC ACID**

(30) Priority: 22.04.2008 JP 2008111725
(71) Applicant: Mitsubishi Gas Chemical Company, Inc., Chiyoda-ku Tokyo 100-8324 (JP)
(72) Inventor: KAWAI, Takeshi, Niigata-shi Niigata 950-3121 (JP); YABUNO, Masashi, Kurashiki-shi Okayama 712-8525 (JP)
(74) Representative: Gille Hrabal Struck Neidlein Prop Roos
(86) International application number: PCT/JP2009/057119
(87) International publication number: WO 2009/130997

(57) **Abstract**

Provided is a process for the production of high-purity trimellitic acid including subjecting a dimethylbenzaldehyde and/or an oxidized derivative of the dimethylbenzaldehyde to liquid-phase oxidation with molecular oxygen in an aqueous solvent containing a catalyst to produce trimellitic acid, in which: 3,4-dimethylbenzaldehyde and/or 3,4-dimethylbenzoic acid are/is used as a raw material; a catalyst containing 0.05 to 1 part by mass of one or more kinds of metals selected from the group consisting of cobalt, manganese, and nickel, 0.0001 to 0.0015 part by mass of metallic iron and/or iron obtained from a water-soluble iron salt, and 1 to 5 parts by mass of bromine with respect to 100 parts by mass of the aqueous solvent is used as the catalyst; and the liquid-phase oxidation is conducted at a temperature of 200 to 250°C. According to the process of the present invention, there can be provided high-quality, high-purity trimellitic acid with extremely small amounts of by-products such as an intermediate of oxidation, an addition compound, and an organobromine compound in high yield. In addition, the residual ratio of bromine used as a catalyst is high, and hence a production cost is curtailed.

## Description

### Technical Field

The present invention relates to a process for the production of high-quality trimellitic acid from a dimethylbenzaldehyde and/or an oxidized derivative of the dimethylbenzaldehyde by continuous liquid-phase air oxidation in high yield in an industrially advantageous manner.

### Background Art

A process involving subjecting pseudocumene to air oxidation in an acetic acid solvent with a cobalt-manganese-bromine-based catalyst has been conventionally known as a production process for trimellitic acid (Patent Literature 1). In addition, the following process has been known (Patent Literature 2). That is, pseudocumene as an oxidation raw material is changed to 2, 4-dimethylbenzaldehyde or 2, 4-dimethylbenzoic acid, and a metal chosen from manganese and cerium, and a bromine compound are used as catalysts upon liquid-phase oxidation of the oxidation raw material with molecular oxygen in an aqueous solvent.

In addition, Patent Literature 3 discloses the following process for the production of trimellitic acid. That is, 2,4-dimethylbenzaldehyde, 2,5-dimethylbenzaldehyde, 3,4-dimethylbenzalci.ehyde, 2,4-dimethylbenzoic acid, 2,5-dimethylbenzoic acid, and/or 3,4-dimethylbenzoic acid are/is used as a raw material, and liquid-phase oxidation is conducted in two stages with molecular oxygen in an aqueous solvent in the presence of a catalyst formed of bromine or of bromine and a heavy metal. In examples in the literature, trimellitic acid is produced from 2, 4-dimethylbenzaldehyde in an aqueous solvent with a catalyst formed of manganese and bromine.
[Patent Literature 1] US 3491144
[Patent Literature 2] JP 56-26839 A
[Patent Literature 3] JP 2002-3440 A

### Summary of Invention

### Technical Problems

In Patent Literature 3 described above, trimellitic acid is produced according to a continuous process and bromine is added at the second stage, and hence the amount of an organobromine compound to be produced as a by-product reduces. However, the amount of the organobromine compound to be produced as a by-product is desirably further reduced because the production of the organobromine compound as a by-product leads to the loss of a bromine component as a catalyst component. In addition, for example, it has been demanded that high-purity trimellitic acid should be produced in additionally high yield by reducing a wastewater treatment load.
An object of the present invention is to provide a process for the industrial production of high-quality, high-purity trimellitic acid in which a dimethylbenzaldehyde and/or an oxidized derivative of the dimethylbenzaldehyde are/is used as a rawmaterial.

### Solution to Problems

The inventors of the present invention have made extensive studies on the above problems. As a result, the inventors have found the following. That is, 3,4-dimethylbenzaldehyde shows behavior completely different from that of 2, 4-dimethylbenzaldehyde used in Examples in Patent Literature 3. When the process described in Patent Literature 3 is applied and 3,4-dimethylbenzaldehyde is used as a raw material, high-purity trimellitic acid cannot be advantageously produced owing to a reduction in yield. However, the addition of a specific amount of iron as a catalyst component enables one to produce high-quality trimellitic acid with small amounts of by-products such as a trimellitic acid bromide in an industrially advantageous manner. Thus, the inventors have reached the present invention.

That is, the present invention provides the following processes for the production of high-purity trimellitic acid:
1. a process for the production of high-purity trimellitic acid including subjecting a dimethylbenzaldehyde and/or an oxidized derivative of the dimethylbenzaldehyde to liquid-phase oxidation with molecular oxygen in an aqueous solvent containing a catalyst to produce trimellitic acid, in which: 3,4-dimethylbenzaldehyde and/or 3,4-dimethylbenzoic acid are/is used as a raw material; a catalyst containing 0.05 to 1 part by mass of one or more kinds of metals selected from the group consisting of cobalt, manganese, and nickel, 0.0001 to 0.0015 part by mass of metallic iron and/or iron obtained from a water-soluble iron salt, and 1 to 5 parts by mass of bromine with respect to 100 parts by mass of the aqueous solvent is used as the catalyst; and the liquid-phase oxidation is conducted at a temperature of 200 to 250°C;
2. the process for the production of high-purity trimellitic acid according to the above item 1, in which the liquid-phase oxidation is conducted according to a two-stage continuous process and 5 to 50 mass% of a total bromine supply amount is supplied at a second stage; and
3. the process for the production of high-purity trimellitic acid according to the above item 1 or 2, in which the aqueous solvent has a mass 1 to 4 times as large as a supply amount of 3,4-dimethylbenzaldehyde and/or 3,4-dimethylbenzoic acid.

### Advantageous Effects of Invention

According to the process of the present invention, high-quality, high-purity trimellitic acid with extremely small amounts of by-products such as an intermediate of oxidation, an addition compound, and an organobromine compound can be obtained from 3,4-dimethylbenzaldehyde and/or 3,4-dimethylbenzoic acid in high yield.
In addition, the residual ratio of bromine used as a catalyst is high. As a result, the replenishment amount of bromine when the catalyst is recycled and reused reduces, and hence a production cost is curtailed.
Further, the discharge amount of by-products such as an organobromine compound to be discharged in a purification step reduces by virtue of the foregoing. As a result, a wastewater treatment load reduces.
Therefore, according to the process of the present invention, high-purity trimellitic acid can be produced in an industrially advantageous manner.

### Description of Embodiments

Hereinafter, the present invention is described in detail.
A large number of kinds including 2,4-dimethylbenzaldehyde, 2,5-dimethylbenzaldehyde, 3,4-dimethylbenzaldehyde, 2,4-dimethylbenzoic acid, 2,5-dimethylbenzoic acid, and 3,4-dimethylbenzoic acid are available as dimethylbenzaldehydes and oxidized derivatives of the dimethylbenzaldehydes each serving as a raw material for the production of trimellitic acid based on liquid-phase oxidation. Of those, 2,4-dimethylbenzaldehyde has been mainly used so far. In the production process of the present invention, in particular, 3,4-dimethylbenzaldehyde and/or 3,4-dimethylbenzoic acid are/is used as a raw material.
In addition, the production process of the present invention is a process for the production of trimellitic acid with air as an oxidant and water as a solvent in which a catalyst obtained by adding iron to a conventional catalyst is used. With the process, trimellitic acid can be produced according to a continuous process.
One of 3,4-dimethylbenzaldehyde and 3,4-dimethylbenzoic acid each serving as a raw material can be used alone, or both of them can be used as a mixture.

One or more kinds of metals selected from the group consisting of cobalt, manganese, and nickel are used in the catalyst of the present invention. Of those, manganese is particularly preferred.
One kind of compounds containing those metals can be used alone, or two or more kinds of them can be used as a mixture. In addition, the compounds containing those metals may each be used in any form, and may each be an organic salt or an inorganic salt. The compounds are desirably present as an ion in the aqueous solvent. The compounds are more preferably bromide salts, or specifically, cobalt bromide, manganese bromide, and nickel bromide.
Bromine used in the catalyst preferably exists as an inorganic bromine compound that generates a bromide ion, and any such metal bromide salt as described above can be used. Hydrobromic acid is more preferably used.

The content of the metal selected from the group consisting of cobalt, manganese, and nickel is 0.05 to 1 part by mass, or preferably 0.1 to 0.5 part by mass with respect to 100 parts by mass of the aqueous solvent. In addition, a bromine content is 1 to 5 parts by mass, or preferably 1.5 to 4 parts by mass with respect to 100 parts by mass of the aqueous solvent.

Metallic iron and/or a water-soluble iron salt are/is used as iron to be added as a catalyst to the aqueous solvent. The water-soluble iron salt, which may be an organic salt or an inorganic salt, is preferably present as an ion in the aqueous solvent. For example, ferric acetate, a ferrous halide, a ferric halide, ferrous sulfate, ferric sulfate, ferrous nitrate, ferric nitrate, and hydrated salts of them can each be used. Of those, ferric bromide is preferred. Only one kind of the metallic iron and/or the water-soluble iron salts may be used, or two or more kinds of them may be used as a mixture. A metallic iron ion eluted from metallic piping is also included.
An iron content is 0.0001 to 0.0015 part by mass, or preferably 0.0002 to 0.0010 part by mass with respect to 100 parts by mass of the aqueous solvent.

A liquid-phase oxidation process is preferably a continuous process. The continuous process is preferably a multi-stage continuous process, or more preferably a two-stage continuous process. A liquid-phase oxidation two-stage continuous process is the following process. A reactor 1 and a reactor 2 are connected in series. An oxidation raw material, a catalyst-containing solvent, and air are loaded from the inlet of the reactor 1, and then liquid-phase oxidation is conducted. A reaction liquid at the outlet of the reactor is delivered to the inlet of the reactor 2. Air is further loaded into the reactor 2, and liquid-phase oxidation is conducted.

In the liquid-phase oxidation two-stage continuous process, it is preferred that bromine be added in two stages, and bromine be added in an amount corresponding to 5 to 50 mass% of the total bromine amount at the second stage to the reactor 2. When bromine is added dividedly as described above, an oxidation rate increases, and an organobromine compound in the system is decomposed so that its amount may reduce. As a result, the amount of an intermediate of oxidation to be produced reduces. Accordingly, trimellitic acid can be obtained in high yield.

Water used as the solvent is preferably, for example, distilled water, ion-exchanged water, or membrane-filtered water, or more preferably water obtained from a water purifying apparatus with a polisher. In addition, the aqueous solvent is used in an amount in the range of preferably 1 to 8 times, or more preferably 1 to 4 times as large as the amount of 3,4-dimethylbenzaldehyde and/or 3,4-dimethylbenzoic acid as a raw material in terms of a mass ratio.

The reaction temperature is 200 to 250°C, or preferably 210 to 230°C. The reaction temperature at each stage in the multi-stage continuous process can be selected from within the temperature range.
Although the reaction pressure is not particularly limited as long as the reaction liquid can be kept in a liquid phase, the reaction pressure typically falls within the range of 1.6 to 6 MPa. The reaction pressure at each stage in the multi-stage continuous process can be selected from within the pressure range.

The process for the production of trimellitic acid of the present invention involves the use of, in particular, 3, 4-dimethylbenzaldehyde and/or 3, 4-dimethylbenzoic acid as a raw material, and is characterized in that the bromide ion residual ratio of the bromine compound used as a catalyst is high.
The term "bromide ion residual ratio" as used herein refers to a ratio at which bromine (in the form of a bromide ion) provided as a catalyst remains in the form of a bromide ion in the aqueous solvent without turning into an organobromine compound as a reaction by-product. The bromide ion serves as a reactant as well as a catalyst. Accordingly, the bromide ion provided for the reaction turns into an organobromine compound at a certain ratio, and the remainder remains as a catalyst having oxidation activity in the aqueous solvent. When the ratio at which the bromide ion remains in the aqueous solvent is high, the recycling of the aqueous solvent to the reaction allows the residual bromide ion to function as a catalyst. As a result, the amount of a bromide ion that must be newly added can be reduced. In other words, a state in which the ratio at which the bromide ion remains in the aqueous solvent after the reaction is high, i.e., a state in which the bromide ion residual ratio is high means that the ratio at which bromine is utilized as a catalyst is high.

To be specific, the above bromide ion residual ratio in the case where 3,4-dimethylbenzaldehyde and/or 3, 4-dimethylbenzoic acid are/is used as a raw material is remarkably high as compared with the bromide ion residual ratio in the case where 2,4-dimethylbenzaldehyde and/or 2,4-dimethylbenzoic acid are/is used as a raw material. In addition, the amount of an organobromine compound in the former case is smaller than that in the latter case. Therefore, the ratio at which the bromide ion can be reused as a catalyst is high. As a result, the replenishment amount of a bromine compound upon reuse of a catalyst liquid can be reduced, and hence high-purity trimellitic acid can be produced in a cost-effective manner.

That is, the process for the production of trimellitic acid of the present invention enables the production of high-quality trimellitic acid excellent in, for example, purity and impurity composition because the amount of an organobromine compound to be produced as a by-product is small, and at the same time, the production of any other impurity can be reduced. In addition, the bromide ion residual ratio of a bromine compound to be used as a catalyst can be increased, and hence high-purity trimellitic acid can be produced in a cost-effective manner. Further, the process is excellent in terms of environmental consciousness because the discharge amount of by-products such as an organobromine compound to be discharged in a purification step reduces by virtue of the foregoing and hence a wastewater treatment load reduces.

### Examples

Next, the present invention is specifically described by way of examples. However, the present invention is not limited by these examples.
A reaction product was analyzed with a gas chromatography apparatus (GC apparatus) under the following conditions in each of the following examples and comparative examples.

| | |
|---|---|
| Model: | Agilent 6890N (manufactured by Agilent Technologies) |
| Column used: | DB-1 (manufactured by Agilent Technologies) |
| Analysis conditions: | Injection Temp 300°C |
| | Detector Temp 300°C |
| Column temperature: | Held at 100°C for 3 minutes increased to 280°C at 5°C/min → held at 280°C for 35 minutes |
| Detector: | Hydrogen flame ionization detector (FID) |
| Analysis method: | First, 1 g of a reaction liquid is precisely weighed and collected in a test tube made of heat-resistant glass, and is then diluted with 3 g of methanol. Further, 3 g of triethylamine hydrochloride and 10 ml of trimethyl phosphate are added to the resultant. The mixed liquid is subjected to a methyl esterification treatment by being heated at 180°C for 40 minutes. |

The treated liquid is cooled to room temperature, and then 20 ml of chloroform are added to the liquid. Further, 0.1 g of triphenylmethane as an internal standard material for GC analysis is precisely weighed, and is then added to and uniformly dissolved in the liquid. After that, 200 ml of water are added to the chloroform solution, and then a liquid-liquid partition process is performed twice. A chloroform layer obtained by leaving the resultant at rest is analyzed with the GC apparatus.

A bromide ion concentration was measured under the following conditions.

| | |
|---|---|
| Titration apparatus: | Automatic potentiometric titrator AT-510 (manufactured by Kyoto Electronics Manufacturing Co., Ltd.) |
| Titrant: | 1/250 normal silver nitrate solution |
| Detection electrodes: | Combined glass electrode C-172 |
| | Silver electrode M-214 |
| | Temperature compensation electrode T-111 |
| Measurement method: | A stirrer made of Teflon (registered trademark) is placed in a 200 ml beaker, and the an appropriate amount of a sample is loaded into the beaker (the mass of the sample is precisely weighed with a balance). Pure water is added to the beaker so that a liquid amount in the beaker may be about 150 ml. Further, about 2 ml of 60mass% nitric acid are added to the beaker. Precipitation titration is performed with the above automatic titrator, and then the bromide ion concentration is determined. |

### Example 1

The liquid-phase air oxidation of 3,4-dimethylbenzaldehyde (3,4-DBAL) was conducted with a continuous two-stage reactor obtained by connecting a reflux condenser, a stirring apparatus, a heating apparatus, and two oxidation reactors made of zirconium each provided with a raw material introducing port, a gas introducing port, and a reaction product discharging port and each having an internal volume of 2 L. Then, 1422 parts by mass of water obtained from a water purifying apparatus with a polisher, 37.3 parts by mass of an aqueous solution of hydrobromic acid (reagent: manufactured by Wako Pure Chemical Industries, Ltd., HBr: 47.0 to 49.0 mass%), 30. 5 parts by mass of MnBr₂·4H₂O (reagent: manufactured by Mitsuwa Chemicals Co., Ltd.), and 0.08 part by mass of a 50 mass% aqueous solution of FeBr₃ (reagent: manufacturedbyMitsuwa Chemicals Co. , Ltd., Fe content: 9.4 mass%) were mixed at the foregoing ratio. Thus, a catalyst liquid A was prepared. In addition, 55.7 parts by mass of water and 4.3 parts by mass of an aqueous solution of hydrobromic acid (reagent: manufactured by Wako Pure Chemical Industries, Ltd., HBr: 47.0 to 49.0 mass%) were mixed at the foregoing ratio. Thus, a catalyst liquid B was prepared.

First, 1490 g of the catalyst liquid A were charged into the reactor at the first stage, and then 1000 g of the catalyst liquid A were charged into the reactor at the second stage. Nitrogen was injected from the gas introducing port of each reactor, and the pressure in the reactor was increased to 1 MPa. In addition, the temperature of each reactor was increased to 220°C with the heating apparatus, and the temperature was maintained during the reaction. Next, 3,4-dimethylbenzaldehyde and the catalyst liquid A were separately supplied at rates of 200 g/h and 750 g/h, respectively to the reactor at the first stage. The inflow of air was initiated from the gas introducing port simultaneously with the supply of 3,4-dimethylbenzaldehyde, and the flow rate was controlled so that an oxygen concentration in a gas discharged from the reactor might be kept at 2.5 vol%. Next, liquid transfer from the reactor at the first stage to the reactor at the second stage was initiated while the liquid surface in the reactor at the first stage was kept constant. At the same time, the catalyst liquid B was supplied at a rate of 60 g/h to the reactor at the second stage, the inflow of air was initiated from the gas introducing port, and the flow rate was controlled so that an oxygen concentration in a gas discharged from the reactor might be kept at 4.5 vol%. A reaction product was extracted from the reactor at the second stage at 1150 g/h while the liquid surface in the reactor at the second stage was kept constant.
During the foregoing, the pressure in the reactor at the first stage was kept at 3.2 MPa, and the pressure in the reactor at the second stage was kept at 2.9 MPa. The amount of the aqueous solvent was 3. 94 times as large as that of 3, 4-dimethylbenzaldehyde in terms of a mass ratio. With regard to the amounts of the respective catalyst components, the amount of bromine was 2.3 parts by mass in total, the amount of manganese was 0.39 part by mass, and the amount of iron was 0.0005 part by mass (5ppm) with respect to 100 parts by mass of water. In addition, the amount of bromine supplied to the reactor at the second stage accounted for 10.5 mass% of the total amount of bromine supplied.

After the composition in each reactor had become stationary, the resultant reaction product liquid was subjected to methyl esterification, and was then subjected to GC analysis. As a result, the yield of trimellitic acid (TMA) was 91%, the yield of a trimellitic acid bromide in which bromine was added to trimellitic acid was 0.1%, the yield of dicarboxymethylbenzenes as intermediates of oxidation in each of which one carboxyl group of trimellitic acid turned into a methyl group (2-methylterephthalic acid and 4-methylisophthalic acid: DCMB) was 0.4%, the yield of trimellides as intermediates of oxidation (1-carboxy-3,4-phthalide and 1-carboxy-4,5-phthalide) was 1.2%, the yield of phthalic acid in which one carboxyl group of trimellitic acid was detached was 0.0%, and the yield of pyromellitic acid (PMA) in which one carboxyl group was added to the aromatic ring of trimellitic acid was 0.1%. In addition, a bromide ion residual ratio was 82%. Table 1 shows the results.

### Example 2

A reaction was conducted in the same manner as in Example 1 except that the catalyst liquid composition was prepared so that the iron content might be 0.0002 part by mass (2 ppm) with respect to 100 parts by mass of water, and a reaction product was subjected to GC analysis. As a result, trimellitic acid was obtained in 89% yield, a trimellitic acid bromide was obtained in 0.1% yield, dicarboxymethylbenzenes were obtained in 0.4% yield, trimellides were obtained in 1.4% yield, phthalic acid was obtained in 0.0% yield, and pyromellitic acid was obtained in 0.2% yield. In addition, a bromide ion residual ratio was 83%. Table 1 shows the results.

### Example 3

A reaction was conducted in the same manner as in Example 1 except that the catalyst liquid composition was prepared so that the iron content might be 0.001 part by mass (10 ppm) with respect to 100 parts by mass of water, and a reaction product was subjected to GC analysis. As a result, trimellitic acid was obtained in 92% yield, a trimellitic acid bromide was obtained in 0.1% yield, dicarboxymethylbenzenes were obtained in 1.1% yield, trimellides were obtained in 2.0% yield, phthalic acid was obtained in 0.1% yield, and pyromellitic acid was obtained in 0.1% yield. Inaddition, a bromide ion residual ratio was 84%. Table 1 shows the results.

### Example 4

A reaction was conducted in the same manner as in Example 1 except that the temperature in each reactor was adjusted to 210°C, and a reaction product was subjected to GC analysis. As a result, trimellitic acid was obtained in 89% yield, a trimellitic acidbromide was obtained in 0.1% yield, dicarboxymethylbenzenes were obtained in 1.2% yield, trimellides were obtained in 3.1% yield, phthalic acid was obtained in 0.0% yield, and pyromellitic acid was obtained in 0.1% yield. In addition, a bromide ion residual ratio was 82%. Table 1 shows the results.

### Example 5

A reaction was conducted in the same manner as in Example 1 except that the total amount of bromine used was 1.5 parts by mass (of which 10.5 mass % was used in the reactor at the second stage), and a reaction product was subjected to GC analysis. As a result, trimellitic acid was obtained in 91% yield, a trimellitic acid bromide was obtained in 0.1% yield, dicarboxymethylbenzenes were obtained in 0.9% yield, trimellides were obtained in 1.8% yield, phthalic acid was obtained in 0.0% yield, and pyromellitic acid was obtained in 0.2% yield. In addition, a bromide ion residual ratio was 79%. Table 1 shows the results.

### Example 6

A reaction was conducted in the same manner as in Example 1 except that 3,4-dimethylbenzoic acid (3,4-DBA) was used instead of 3, 4-dimethylbenzaldehyde as a rawmaterial, and a reaction product was subjected to GC analysis. As a result, trimellitic acid was obtained in 93% yield, a trimellitic acid bromide was obtained in 0.1% yield, dicarboxymethylbenzenes were obtained in 0.4% yield, trimellides were obtained in 1.5% yield, phthalic acid was obtained in 0.0% yield, and pyromellitic acid was obtained in 0.1% yield. In addition, a bromide ion residual ratio was 86%. Table 1 shows the results.

### Comparative Example 1

A reaction was conducted in the same manner as in Example 4 except that the 50 mass% aqueous solution of FeBr₃ was not used, and a reaction product was subjected to GC analysis. As a result, trimellitic acidwas obtained in 66% yield, a trimellitic acidbromide was obtained in 0.2% yield, dicarboxymethylbenzenes were obtained in 0.8% yield, trimellides were obtained in 4.4% yield, phthalic acid was obtained in 0.1% yield, and pyromellitic acid was obtained in 0.1% yield. In addition, a bromide ion residual ratio was 74%. Table 2 shows the results.

### Comparative Example 2

A reaction was conducted in the same manner as in Example 4 except that the catalyst liquid composition was prepared so that the iron content might be 0.0020 part by mass (20 ppm) with respect to 100 parts by mass of water, and a reaction product was subjected to GC analysis. As a result, trimellitic acid was obtained in 86% yield, a trimellitic acid bromide was obtained in 0.1% yield, dicarboxymethylbenzenes were obtained in 1.4% yield, trimellides were obtained in 3.9% yield, phthalic acid was obtained in 0.1% yield, and pyromellitic acid was obtained in 0.4% yield. Inaddition, a bromide ion residual ratio was 76%. Table 2 shows the results.

### Comparative Example 3

A reaction was conducted in the same manner as in Example 1 except that the oxidation raw material was changed to 2,4-dimethylbenzaldehyde (2,4-DBAL), and a reaction product was subjected to GC analysis. As a result, trimellitic acid was obtained in 87% yield, a trimellitic acid bromide was obtained in 0.9% yield, dicarboxymethylbenzenes were obtained in 0.3% yield, trimellides were obtained in 1.2% yield, phthalic acid was obtained in 0.0% yield, and pyromellitic acid was obtained in 0.1% yield. In addition, a bromide ion residual ratio was 39%. Table 2 shows the results.

### Comparative Example 4

A reaction was conducted in the same manner as in Example 3 except that the oxidation raw material was changed to 2,4-dimethylbenzaldehyde, and a reaction product was subjected to GC analysis. As a result, trimellitic acid was obtained in 83% yield, a trimellitic acid bromide was obtained in 0.9% yield, dicarboxymethylbenzenes were obtained in 0.7% yield, trimellides were obtained in 2.0% yield, phthalic acid was obtained in 0.0% yield, and pyromellitic acid was obtained in 0.2% yield. In addition, a bromide ion residual ratio was 34%. Table 2 shows the results.

### Comparative Example 5

Liquid-phase air oxidation was conducted in the same manner as in Comparative Example 3 except that the 50 mass% aqueous solution of FeBr₃ was not used. As a result, trimellitic acid was obtained in 91% yield, a trimellitic acid bromide was obtained in 0.8% yield, dicarboxymethylbenzenes were obtained in 0.4% yield, trimellides were obtained in 2.1% yield, phthalic acid was obtained in 0.1% yield, and pyromellitic acid was obtained in 0.1% yield. In addition, a bromide ion residual ratio was 56%. Table 2 shows the results.

[Table 1]

**Table 1**

| | Example 1 | Example 2 | Example 3 | Example 4 | Example 5 | Example 6 |
|---|---|---|---|---|---|---|
| Raw material | 3,4-DBAL | 3,4-DBAL | 3,4-DBAL | 3,4-DBAL | 3,4-DBAL | 3,4-DBA |
| Catalyst component concentration (mass per 100 parts by mass of water) | | | | | | |
| Mn | 0.39 | 0.39 | 0.39 | 0.39 | 0.39 | 0.39 |
| Fe | 0.0005 | 0.0002 | 0.0010 | 0.0005 | 0.0005 | 0.0005 |
| Br | 2.3 | 2.3 | 2.3 | 2.3 | 1.5 | 2.3 |
| Reaction temperature (°C) | 220 | 220 | 220 | 210 | 220 | 220 |
| Yield (%) | | | | | | |
| Trimellitic acid | 91 | 89 | 92 | 89 | 91 | 93 |
| Trimellitic acid bromide | 0.1 | 0.1 | 0.1 | 0.1 | 0.1 | 0.1 |
| Dicarboxymethylbenzenes | 0.4 | 0.4 | 1.1 | 1.2 | 0.9 | 0.4 |
| Trimellides | 1.2 | 1.4 | 2.0 | 3.1 | 1.8 | 1.5 |
| Phthalic acid | 0.0 | 0.0 | 0.1 | 0.0 | 0.0 | 0.0 |
| Pyromellitic acid | 0.1 | 0.2 | 0.1 | 0.1 | 0.2 | 0.1 |
| Unidentified component | 7.2 | 8.9 | 4.6 | 6.5 | 6.0 | 4.9 |
| Bromide ion residual ratio in reaction product liquid (%) | 82 | 83 | 84 | 82 | 79 | 86 |

[Table 2]

**Table 2**

| | Comparative Example 1 | Comparative Example 2 | Comparative Example 3 | Comparative Example 4 | Comparative Example 5 |
|---|---|---|---|---|---|
| Raw material | 3,4-DBAL | 3,4-DBAL | 2,4-DBAL | 2,4-DBAL | 2,4-DBAL |
| Catalyst component concentration (mass per 100 parts by mass of water) | | | | | |
| Mn | 0.39 | 0.39 | 0.39 | 0.39 | 0.39 |
| Fe | 0.0000 | 0.0020 | 0.0005 | 0.0010 | 0.0000 |
| Br | 2.3 | 2.3 | 2.3 | 2.3 | 2.3 |
| Reaction temperature (°C) | 210 | 220 | 220 | 220 | 220 |
| Yield (%) | | | | | |
| Trimellitic acid | 66 | 86 | 87 | 83 | 91 |
| Trimellitic acid bromide | 0.2 | 0.1 | 0.9 | 0.9 | 0.8 8 |
| Dicarboxymethylbenzenes | 0.8 | 1.4 | 0.3 | 0.7 | 0.4 |
| Trimellides | 4.4 | 3.9 | 1.2 | 2.0 | 2.1 |
| Phthalic acid | 0.1 | 0.1 | 0.0 | 0.0 | 0.1 |
| Pyromellitic acid | 0.1 | 0.4 | 0.1 | 0.2 | 0.1 |
| Unidentified component | 28.4 | 8.1 | 10.5 | 13.2 | 5.5 |
| Bromide ion residual ratio in reaction product liquid (%) | 74 | 76 | 39 | 34 | 56 |

It should be noted that, in Tables 1 and 2, 3, 4-DBAL, 2,4-DBAL, and 3,4-DBA as raw materials represent 3,4-dimethylbenzaldehyde, 2,4-dimethylbenzaldehyde, and 3,4-dimethylbenzoic acid, respectively.
A catalyst component concentration is the mass of a catalyst component per 100 parts by mass of the aqueous solvent, the yield of trimellitic acid or the like is a molar ratio (%) of trimellitic acid or the like obtained with respect to the above raw material, and the bromide ion residual ratio is a molar ratio (%) of the bromide ion in a reaction product liquid to bromine (bromide ion) supplied as a catalyst component as described above.
1-carboxy-3, 4-phthalide as one of the trimellides is a compound represented by the following chemical structural formula on the right side.

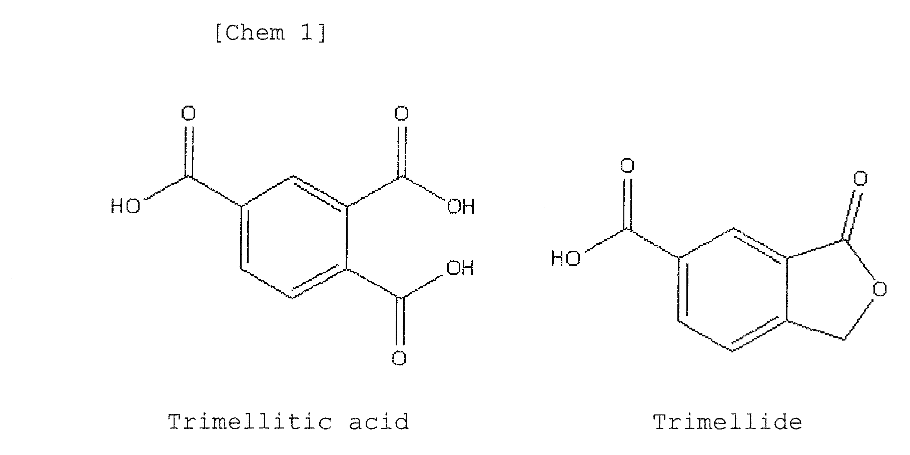

Tables 1 and 2 show the following.
(1) As can be seen from Examples 1 to 5 and Comparative Example 1, when iron is not added to a catalyst upon liquid-phase air oxidation of 3, 4-dimethylben.zaldehyde as a raw material in an aqueous solvent according to a continuous process unlike the present invention, the yield of trimellitic acid does not reach 70%, and hence a satisfactory yield cannot be obtained. However, when iron is added at a concentration of 2 ppm, 5 ppm, or 10 ppm, trimellitic acid canbe obtained in about 90% yield, which is an industrially sufficient yield.

(2) As can be seen from Comparative Examples 3 to 5, when 2,4-dimethylbenzaldehyde that has been conventionally used is used as a raw material, the best yield is achieved in the case where iron is not added, and the yield reduces as the amount of iron increases.

(3) When Example 1 and Comparative Example 5 in which the same trimellitic acid yield was obtained are compared, the use of 3,4-dimethylbenzaldehyde as a raw material like the present invention enables the production of extremely high-purity trimellitic acid with small amounts of a trimellitic acid bromide, trimellides, phthalic acid, and the like as compared with that obtained in the case where 2,4-dimethylbenzaldehyde as a conventional raw material is used.

(4) When 3,4-dimethylbenzaldehyde is used as a raw material like the present invention, the residual ratio of a bromide ion that can be utilized as a catalyst is high, and hence the amount of a bromine compound to be newly added upon recycling and reuse of an aqueous solvent can be reduced. For example, in the case where 2,4-dimethylbenzaldehyde and/or 2,4-dimethylbenzoic acid as a conventional raw material are/is subjected to liquid-phase air oxidation in the aqueous solvent according to a continuous process, a bromide ion residual ratio obtained by comparing bromide ion amounts before and after the reaction is about 50%. In contrast, in the case of 3,4-dimethylbenzaldehyde and/or 3,4-dimethylbenzoic acid of the present application on condition that trimellitic acid is obtained in the same yield as that in the above case, the bromide ion residual ratio is as high as about 80%, and hence trimellitic acid can be produced in a cost-effective manner.

(5) With regard to the production of by-products, in particular, when the amount of a trimellitic acid bromide as an organobromine compound to be produced in the case where 2,4-dimethylbenzaldehyde and/or 2,4-dimethylbenzoic acid as a conventional raw material are/is used is set to 1, the amount of the trimellitic acid bromide in the case where 3,4-dimethylbenzaldehyde and/or 3,4-dimethylbenzoic acid of the present invention are/is used as a raw material reduces to 0.11 to 0.125 (1/9 to 1/8).

(6) In addition, when the amount of a trimellide as an intermediate of oxidation to be produced in the case where 2,4-dimethylbenzaldehyde and/or 2,4-dimethylbenzoic acid as a conventional raw material are/is used is set to 1, the amount in the case where 3, 4-dimethylbenzaldehyde and/or 3, 4-dimethylbenzoic acid of the present invention are/is used as a raw material reduces to 0.5 to 1 (1/2 to 1/1).

(7) Further, in the case where 3, 4-dimethylbenzaldehyde and/or 3,4-dimethylbenzoic acid of the present invention are/is used as a raw material, the amounts of by-products and intermediates of oxidation remarkably reduce. For example, in this case, phthalic acid to be produced in the case where 2, 4-dimethylbenzaldehyde and/or 2, 4-dimethylbenzoic acid as a conventional raw material are/is used is scarcely produced. As a result, high-quality, high-purity trimellitic acid can be produced. In particular, a reduction in amount of phthalic acid to be produced leads to the reduction of the production of dioctyl phthalate (DOP) upon production of a plasticizer (trioctyl trimellitate: TOTM) by the esterification of trimellitic acid with octyl alcohol.

Therefore, behavior in the case where 3,4-dimethylbenzaldehyde and/or 3,4-dimethylbenzoic acid are/is used as a raw material like the present invention is different from that in the case where 2,4-dimethylbenzaldehyde and/or 2,4-dimethylbenzoic acid are/is used, and the addition of iron to a catalyst not only remarkably improves the yield of trimellitic acid but also provides extremely high-purity trimellitic acid. In addition, the bromide ion residual ratio is as high as about 80%, and hence trimellitic acid can be produced in a cost-effective manner. Accordingly, the production process of the present invention is found to be a production process excellent in terms of environmental consciousness.

## Claims

1. A process for production of high-purity trimellitic acid, he process comprising subjecting a dimethylbenzaldehyde and/or an oxidized derivative of the dimethylbenzaldehyde to liquid-phase oxidation with molecular oxygen in an aqueous solvent containing a catalyst to produce trimellitic acid, wherein:
3,4-dimethylbenzaldehyde and/or 3,4-dimethylbenzoic acid are/is used as a raw material;
a catalyst containing 0.05 to 1 part by mass of one or more kinds of metals selected from the group consisting of cobalt, manganese, and nickel, 0.0001 to 0.0015 part by mass of metallic iron and/or iron obtained from a water-soluble iron salt, and 1 to 5 parts by mass of bromine with respect to 100 parts by mass of the aqueous solvent is used as the catalyst; and
the liquid-phase oxidation is conducted at a temperature of 200 to 250°C.

2. The process for production of high-purity trimellitic acid according to claim 1, wherein:
the liquid-phase oxidation is conducted according to a two-stage continuous process; and
5 to 50 mass% of a total bromine supply amount is supplied at a second stage.

3. The process for production of high-purity trimellitic acid according to claim 1 or 2, wherein the aqueous solvent has a mass 1 to 4 times as large as a supply amount of 3,4-dimethylbenzaldehyde and/or 3,4-dimethylbenzoic acid.
